# EUROPEAN PATENT APPLICATION

(11) **EP 3 213 646 A1**
(43) Date of publication of application: **06.09.2017**
(21) Application number: 17159013.6
(22) Date of filing: 02.03.2017
(51) Int. Cl.: A23L 33/105, A21D 2/36, A21D 13/06, A61K 36/63, A23L 19/00

(54) **METHOD FOR THE PRODUCTION OF OLIVE FLOUR SUITABLE AS A FOOD SUPPLEMENT**

(30) Priority: 03.03.2016 EP 16158560
(71) Applicant: OLIOCRU' S.r.l., 38062 Arco (TN) (IT)
(72) Inventor: CONTERNO, Lorenza, 38062 Arco (TN) (IT); MARTINELLI, Francesca, 38062 Arco (TN) (IT); MORANDINI, Marco, 38062 Arco (TN) (IT); TAMBURINI, Matteo, 38062 Arco (TN) (IT)
(74) Representative: Laghi, Alberto

(57) **Abstract**

Process for the production of a food supplement, characterized in that it comprises the following operational phases:- arranging a certain quantity of olives containing a predetermined amount of polyphenols;- subjecting said quantity of olives to a grinding operation and consequently separation of the oil obtained by said grinding so as to obtain an oil-free olive paste;- subjecting said olive paste devoid of oil to a drying operation to obtain a dried paste;- subjecting said dried pasta of olives to a grinding operation and subsequent sieving to obtain a flour of olives; - and introducing said flour in capsules, tablets or sachets, or the like.

## Description

The present invention relates to a process for the production of a food supplement.

In particular, the present invention is to be used as advantage for the food industry to produce a food supplement through the processing of a food grade paste of olives or equivalent one seed fruits, to which the description hereafter will make explicit reference without thereby losing in generality.

As it is known, the olives contain a number of substances, such as polyphenols, tocopherols, sterols, squalene, etc., which have been shown to play a very important and significant role for human health.

In general, only a small percentage of these substances is transferred to the oil during the olive oil production.

In fact, the polyphenols, substances with high antioxidant capacity, represent the 1-2% of the fresh weight of the olive, while in the oil it is possible to find about the 0.1 % at most; fibres naturally present in the olive fruit are not transferred to the oil because of their not fat soluble nature.

The purpose of the present invention is therefore to provide a process to produce a food supplement with high content of polyphenols.

The functional characteristics of the present invention and its advantages over the state of the art, will be highlighted and clarified after the claims hereafter, and in particular after the examination of the description, referring to the flow chart annexed to this document showing the preferred, but not the limiting variant, of the production process of the food supplement in question.

The starting raw material is a given amount of olives collected in the optimal seasonal period (depending on the variety) established to obtain the necessary amount of polyphenols (determined through in vivo study).

The whole olives are ground by crushing or, preferably, by destoning, to obtain a food grade paste of milled olives.

In particular, it has to be highlighted that destoning, unlike the most common process in which the olive pulp is ground together with the pit (crushing), effectively allows to obtain olive paste of higher quality, free of those enzymes present in the seeds and released into the paste from the broken pits. These enzymes are, together with the oxygen of the air, degradation agents of the paste polyphenols (oxidation).

Moreover, the absence of pit fragments, otherwise generated during crushing, makes the paste suitable for the human consumption without other treatment.

In the next step the olive paste is separated from the oil, whose presence would lead to both a technological problem in the later step of drying and a higher unwanted energy content in the final product.

To allow the olive paste thus obtained to be used as food supplement, such olive paste is to be subjected to a drying step and then be ground to obtain a powder. This olive flour, for commercial purpose, can be placed in capsules, single-dose sachets, tablets or similar.

According to the flow chart in the attached diagram, the drying process takes place at low temperature under vacuum (less than 60°C), or by microwave treatment, or again by exploiting other technologies (for example without vacuum and at temperatures higher than 60°C) to avoid oxidation during the drying phase.

As mentioned above, the dried product is ground and sieved to avert the risk of accidental presence of pit fragments, and finally the olive flour obtained is introduced into capsules, single-dose sachets, tablets or similar through known processes.

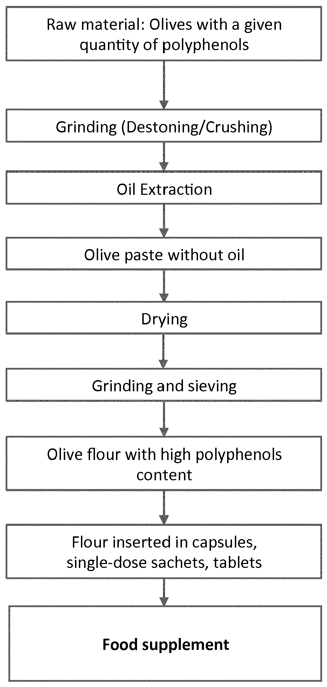

The prebiotic ability of olive flour have already been experimentally verified in 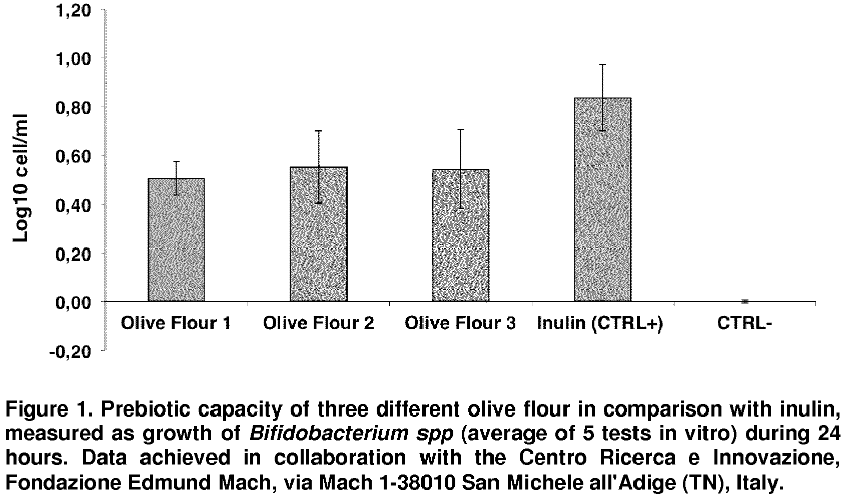 vitro, as Figure 1 shows. The olive flour in fact, in an in vitro system used as a model of the human intestinal microflora activity, has shown the capacity to induce the growth of bifidobacteria similarly to a positive control (inulin, standard prebiotic).

### DEFINITIONS:

Destoning: grinding the olives with a special machine (destoner) that separates the whole pits of the olives while mashing the fruit pulp.
Crushing: grinding the olive the traditional way, pulp and pits all together (can be done with a variety of machines: hammer mills, stone mills, metal tooth grinders).

## Claims

1. Process for the production of a food supplement, **characterized in that** it comprises the following operational phases:
- arranging a certain quantity of olives containing a predetermined amount of polyphenols;
- subjecting said quantity of olives to a grinding operation and consequently separation of the oil obtained by said grinding so as to obtain an oil-free olive paste;
- subjecting said olive paste devoid of oil to a drying operation to obtain a dried paste;
- subjecting said dried pasta of olives to a grinding operation and subsequent sieving to obtain a flour of olives;
- and introducing said flour in capsules, tablets or sachets, or the like.

2. A process according to claim 1 **characterized in that** said drying step is carried out under vacuum and occurring at a temperature below 60°C.

3. A process according to claim 1 **characterized in that** said drying step is carried out by microwave treatment.

4. A process according to any of claims 1 to 3 **characterized in that** said drying step is carried out at a temperature above 60°C.

5. Food supplement made with the process according to any of the previous claims 1 to 4.
